Europäisches Patentamt

European Patent Office

Office européen des brevets

(19)

(11) **EP 1 007 357 B1**

(12) **EUROPEAN PATENT SPECIFICATION**

(45) Date of publication and mention
of the grant of the patent:
**04.08.2004 Bulletin 2004/32**

(51) Int Cl.⁷: **B32B 31/12**, B32B 7/04,
B32B 3/10

(21) Application number: **97931079.4**

(22) Date of filing: **06.06.1997**

(86) International application number:
**PCT/US1997/009947**

(87) International publication number:
**WO 1998/055298 (10.12.1998 Gazette 1998/49)**

(54) **METHODS FOR FORMING EXTENSIBLE LAMINATE STRUCTURES**

VERFAHREN ZUM FORMEN VON DEHNBAREN SCHICHTSTOFFSTRUKTUREN

TECHNIQUES DE PRODUCTION DE STRUCTURES LAMINEES EXTENSIBLES

(84) Designated Contracting States:
**AT BE CH DE DK ES FI FR GB GR IE IT LI LU NL
PT SE**

(43) Date of publication of application:
**14.06.2000 Bulletin 2000/24**

(73) Proprietor: **THE PROCTER & GAMBLE COMPANY
Cincinnati, Ohio 45202 (US)**

(72) Inventors:
• **LANGDON, Frederick, Michael
Ashiya 659 (JP)**
• **HAMMOND, Keith, Waymon
Hamilton, OH 45011 (US)**

(74) Representative: **Hirsch, Uwe Thomas et al
Procter & Gamble European Service GmbH,
Sulzbacher Strasse 40-50
65824 Schwalbach am Taunus (DE)**

(56) References cited:
**EP-A- 0 023 804        EP-A- 0 346 928
WO-A-95/19258        GB-A- 1 367 959
US-A- 5 407 507**

• **Encyclopedia of Polymer Science and
Engineering, vol. 1 (1985); John Wiley & Sons,
New York (US); p. 549**
• **I. Skeist: 'Handbook of Adhesives', 2nd ed.
(1977); Van Nostrand Reinhold Co., New York
(US); p 101**

**Description**

FIELD

The present invention relates generally to the field of methods for forming laminate structures. More particularly, the present invention relates to methods for forming extensible laminate structures that can be incorporated into disposable absorbent garments.

BACKGROUND

Laminate structures have previously been used in a variety of products, including elastic absorbent articles such as sweat bands, bandages, diapers and incontinence devices. Examples of laminate structures that have been previously used include composites made from a gatherable material joined to an elastic web, composites made from mesh, tissue and microfibers, as well as composites made from a central elastic layer, a breathable material, and an adhesive layer. Examples of such laminates are described in U.S. Patent No. 4,522,863 to Keck et al. and U.S. Patent No. 4,977,011 to Smith.

WO 95/19258 discloses a stretch-activated elastic composite comprising a non-woven fabric having a potential elongatibility of higher than 100 % in a predetermined direction and an elastically recoverable elastic sheet, which, in its unstretched state, is partially bonded to the non-woven fabric in its unelongated state.

Laminate structures have been used in disposable pull-on garments that are donned by inserting the wearer's legs into the leg openings and sliding the garment up into position about the lower torso. Examples of such pull-on garments include disposable underwear, pull-on diapers, training pants, and disposable panties for menstrual use. Such garments are often worn by infants and other incontinent individuals to receive and contain urine and other body exudates. Examples of these types of pull-on garments are disclosed in U.S. Patent No. 5,171,239 to Igaue et al., U.S. Patent No. 4,610,681 to Strohbeen et al., WO 93/17648 published on September 16, 1993, U.S. Patent No. 4,940,464 to Van Gompel et al., U.S. Patent No. 5,246,433 to Hasse et al., and U.S. Patent No. 5,569,234 to Buell et al.

Disposable pull-on garments having fixed sides (e.g., training pants or pull-on diapers) have become popular for use on children able to walk and often who are toilet training. In order to contain body exudates as well as to fit a wide variety of body shapes and sizes, these pants must fit snugly about the waist and legs of the wearer without drooping, sagging or sliding down from position on the torso, as well as accommodate larger wearers without causing irritation to the skin due to the product being too tight. Thus, the pant must have elastic extensibility in the waist and legs with the elastic features providing a high degree of stretch. Extensible side panels, including side panels formed from a stretch laminate, have previously been used to provide a degree of stretchiness in the side areas of such garments.

However, pull-on garments provided with conventional extensible side panels typically, when under stretch, have a tendency to sag, droop, or slide down the wearer's body. This is particularly true if the extensible side panels have been formed from a laminate structure. When strained over an interval of time, i.e., during the wearing cycle (the time period that the garment is worn), the layers of the laminate structure may move relative to one another. Such relative movement is known as "creep" and generally results in reduction of the holding forces generated in the side panel. In addition, the layers of the laminate structure may become delaminated under shear forces generated during the wearing cycle, which also causes the holding forces generated in the side panels to decrease. Such reduction in these holding forces typically leads to sag, droop, or sliding down, and thus the likelihood that body exudates may not be adequately contained and leakage may occur. In addition, a garment that experiences sag, droop or sliding down is generally less comfortable for the wearer and unacceptable to the consumer.

In addition, conventional side panels have generally required trade-offs between performance characteristics, such as breathability, soft feel for the wearer, application force, and sustained contractive forces. In order to improve performance in one area, performance in another area has typically been compromised. For example, conventional side panels requiring a high degree of stretch at application of a diaper (e.g., about 200%) require low application forces to be acceptable to the consumer. However, at lower levels of side-panel stretch that are typical during the wearing cycle (e.g., about 50%) such side panels exhibit sustained contractive forces that are too low to prevent diaper sag.

Additionally, conventional side panels that can strain up to 150%-200% during a diaper wear cycle typically create shear forces upon the laminated elastic elements that cause delamination and creep from the fabric or material elements.

Based on the foregoing, there is a need for an extensible laminate structure, and methods for forming the same, that substantially eliminates creep and delamination, thereby substantially eliminating sagging, drooping, and sliding down of a garment that incorporates such a laminate during wear, and which necessitates no performance trade-offs. None of the existing laminate structures or pull-garments provide all of the advantages and benefits of the present invention.

SUMMARY

The present invention is directed to methods according to claim 1 for forming a laminate structure elastically extensible in at least one direction, comprising providing a first coverstock layer; providing a second coverstock layer; providing an elastomeric layer between the first and the second coverstock layers; and joining the elastomeric layer between the first and second coverstock layers and along edge regions thereof to provide side anchor zones along edge regions of the laminate structure.

These and other features, aspects, and advantages of the invention will become evident to those skilled in the art from a reading of the present disclosure.

BRIEF DESCRIPTION OF THE DRAWINGS

While the specification concludes with claims particularly pointing out and distinctly claiming the invention, it is believed that the present invention will be better understood from the following description which is taken in conjunction with the accompanying drawings, in which like designations are used to designate substantially identical elements, and in which:

Figure 1 is an exploded view of the layers of a preferred embodiment of the laminate structure of the present invention prior to being formed into a laminate structure;

Figures 2A and 2B are fragmentary side views of a laminate structure made in accordance with the present invention;

Figures 3A-3C are enlarged views of a portion of the laminate structure of Figure 2 at various stages during the formation of the laminate structure;

Figure 4 is a top view of a portion of the laminate structure shown in Figures 1-3;

Figure 5 is a schematic representation of a preferred process for forming the laminate structure of Figures 1-4;

Figure 6 is a perspective view of a typical disposable pull-on garment incorporating the laminate structure of the present invention in a typical in-use configuration; and

Figure 7 is a cross-sectional perspective view of a portion of the pull-on garment shown in Figure 6.

DETAILED DESCRIPTION

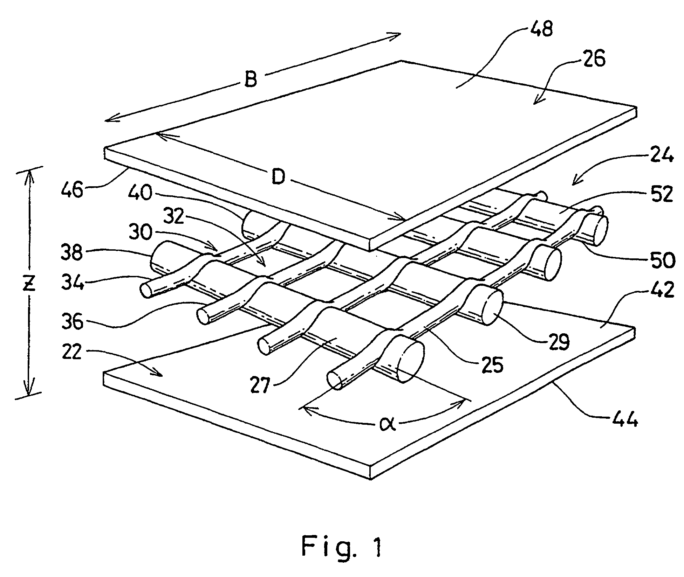

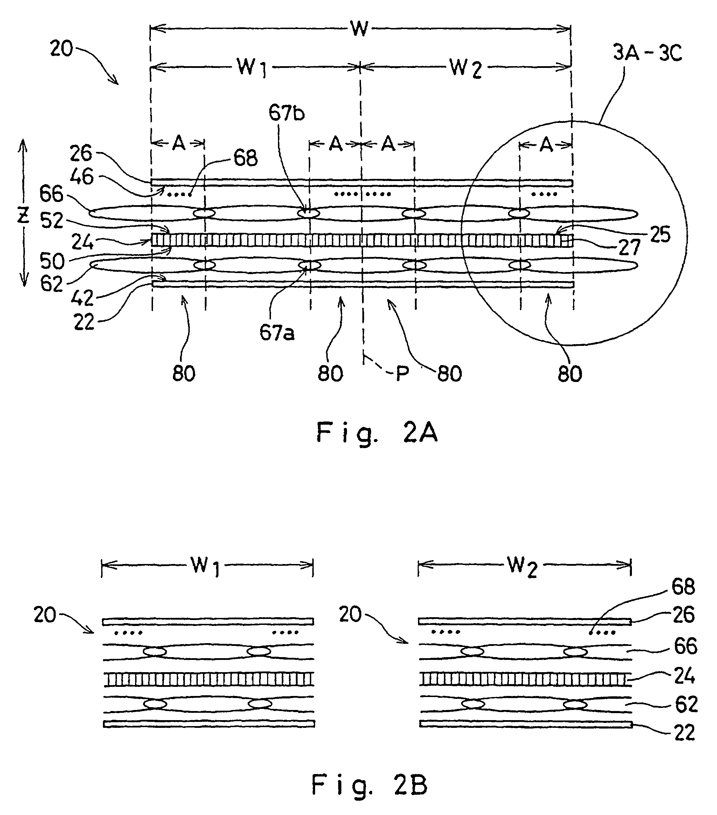

Figure 1 is an exploded view of the components of a preferred embodiment of the laminate structure 20 prior to formation according to the methods of the present invention (a preferred embodiment of laminate structure 20 after formation is shown in side view in Figure 2). The laminate structure 20 is formed from a first coverstock layer 22, a elastomeric layer 24 that has a plurality of first strands 25 and a plurality of second strands 27, and a second coverstock layer 26.

The laminate structure 20 has at least one structural direction D, wherein at least a portion of the structural direction D is elastically extensible. More preferably, the laminate structure 20 provides the structural direction D along the direction and entire length of the second strands 27. A structural direction B is provided along the direction and entire length of the first strands 25 and is substantially perpendicular to the structural direction D. A structural direction z is further provided and is defined as the direction extending through the thickness of the laminate 20.

Although it is preferred that the laminate structure 20 provide at least one structural direction D that is elastically extensible, it is further contemplated that the laminate structure 20 can be inelastic such that no elastically extensible structural directions are provided. Alternatively, the laminate structure 20 can also be provided with a structural direction over which a portion of the length thereof is elastically extensible and a portion of the length thereof is inelastic. As used herein, "elastically extensible" means a segment or portion that will elongate in at least one direction (preferably the lateral or structural direction D) when tensional forces are applied, and will return to about its previous size and configuration when the tensional forces are removed.

Referring to Figure 1, the elastomeric layer 24 is provided with a plurality of first strands 25 that intersect a plurality of second strands 27 at nodes 30 at a predetermined angle a, forming a net-like open structure having a plurality of apertures 32. Each aperture 32 is defined by at least two adjacent first strands (e.g., 34 and 36) and at least two adjacent second strands (e.g., 38 and 40), so that the apertures 32 are substantially rectangular in shape. Other configurations of the apertures 32, such as parallelograms, squares, or circular arc segments, can also be provided. Preferably, the first and second strands 25 and 27 are substantially straight and substantially parallel to one another. Preferably, the first strands 25 intersect the second strands 27 at nodes 30 such that the angle $\alpha$ is about 90 degrees. The first and second strands 25 and 27 are preferably joined or bonded at nodes 30.

Preferably, the elastomeric layer 24 is an elastomeric scrim material. A preferred elastomeric scrim is manufactured by the Conwed Plastics Company under the designation TN2514. This material has 4.7 elastic strands per centimeter (12 elastic strands per inch) in the structural direction B (i.e., the first strands 25) and 2.8 elastic strands per centimeter (7 elastic strands per inch) in the structural direction D (i.e., the second strands 27). Other preferred materials for use as the elastomeric layer 24 include perforated films and elastic strands, where the elastic strands may be laid in either or both of the structural directions B and D, or may run in a continuous linear fashion across the coverstock layer from side to side (creating a continuous elongated "S" type pattern). Alternatively, other elastomeric materials such as "live" synthetic or natural rubber, other synthetic or natural rubber foams, elastomeric films (including heat shrinkable and apertured elastomeric films), elastomeric woven or nonwoven webs, elastomeric composites, or other such materials as are known to the artisan may be used. However, typical film elastic materials and other elastomeric layers tend to occlude vapor transmission through the laminate structure, which may decrease breathability.

The first coverstock layer 22 has an inner surface 42 and an outer surface 44. The inner surface 42 of the first coverstock layer 22 is the surface that is positioned facing the elastomeric layer 24. The second coverstock layer 26 also has an inner surface 46 and an outer surface 48. The inner surface 46 of the second coverstock layer 26 is the surface that is positioned facing the elastomeric layer 24. The elastomeric layer 24 also has two planar surfaces, first surface 50 and second surface 52 (more clearly shown in Figure 2A), each of which is substantially parallel with the planes of the first and second coverstock layers 22 and 26. The first surface 50 is that planar surface of the elastomeric layer 24 that is most closely adjacent with the inner surface 42 of first coverstock layer 22. The second surface 52 is that planar surface of elastomeric layer 24 that is most closely adjacent to the inner surface 46 of the second coverstock layer 26.

Since the laminate structure 20 will be subjected to mechanical stretching during use, the first and second coverstock layers 22 and 26 are preferably elongatable, more preferably drawable (but not necessarily elastomeric), without undue, and preferably without any, tearing or ripping. Further, because the typical uses contemplated for the laminate 20 involve incorporation into articles that will contact a wearer's skin, the first and second coverstock layers 22 and 26 are preferably compliant, soft feeling, and nonirritating to the wearer's skin and give the article the feel and comfort of a cloth garment. Suitable materials can be manufactured from a wide range of materials such as plastic films, apertured plastic films, woven or nonwoven webs of natural fibers (e.g., wood or cotton fibers), synthetic fibers (e.g., polyolefins, polyamides, polyester, polyethylene, or polypropylene fibers), or a combination of natural and/or synthetic fibers, or coated woven or nonwoven webs.

Preferably, each of the first and second coverstock layers 22 and 26 is an identical consolidated nonwoven material. An exemplary preferred nonwoven material is manufactured by the FiberWeb Company under the designation Sofspan 200. This material has a basis weight of 25 g/m$^2$ before consolidation. As used herein, "basis weight" is the weight of one square meter of planar web material. Alternatively, highly strainable nonwoven materials may be used. Alternatively, the first and second coverstock layers 22 and 26 need not be of identical materials, as long as the desired performance requirements, such as elastic performance, softness, flexibility, breathability and durability, are met. As used herein, "consolidated nonwoven material" means a nonwoven material that has been gathered or necked under mechanical tension in the structural direction D so that the material can elongate in the structural direction D under low force.

The laminate structure 20 is preferably formed by positioning the elastomeric layer 24 between the first coverstock layer 22 and the second coverstock layer 26, as shown in Figure 1, and adhesively bonding the three layers using the bond patterns as described below. Although preferred embodiments of the present invention are described herein with reference to a laminate having three layers it will be apparent to those of skill in the art that the laminate structures of the present invention are not limited to those having three layers. In addition, it will further be apparent to those of skill in the art that the side anchor zones of the laminates of the present invention are not limited to those having been formed from one, two, or three adhesive applications, but rather may be formed by any means known to the artisan.

Thus, although variations in the number, type and order of application of the adhesives that are used to form the laminates of the present invention are within the scope of the present invention, preferably at least a first adhesive is applied to the inner surface of at least one of the coverstock layers to create the side anchor zones A along edge regions of the laminate structure 20, as described more fully below.

A fragmentary enlarged side view looking into the structural direction B of the laminate 20 is shown in Figure 2A. It has been found that when the laminate 20 is bonded or otherwise anchored such that side anchor zones A are created, such a laminate 20 is both highly elastic and substantially free from delamination and creep, while providing very good performance characteristics in all performance categories with no trade-offs between any performance characteristics required. The side anchoring is preferably performed by side gluing with adhesive beads to anchor the elastomeric layer between the coverstock layers as a part of the lamination process, as described more fully below. Alternatively, side anchoring may be performed by sewing, heat sealing, ultrasound bonding, needle punching, alternative gluing processes, or by any other means known to those skilled in the art. Another alternative is to side anchor the layers of the laminate structure after the lamination of the elastomeric and coverstock components has been performed.

The laminates of the present invention may particularly provide very good soft feel for the wearer and for the consumer. This is important because consumers value softness. In conventional laminates, the attempts to eliminate creep have frequently required an unacceptable decrease in softness, often accompanied by an unacceptable decrease in the ability to activate. This is because such previous attempts (which have fallen short of eliminating creep) have focused on the application of additional melt blown adhesive, often in an overall coating pattern, in the attempt to strengthen the bonds. This has generally resulted in an undesirable overall stiffening of the laminate. However, the laminates of the present invention provide elimination of creep without the loss of consumer-desired soft feel and without compromise of activation ability.

Referring to Figure 2A, a first adhesive 70 (not separately shown in the Figures) is applied to the inner surface 46 of the second coverstock layer 26 in positions that correspond to each of the outer edges 80 of the laminate structure 20. The first adhesive 70 may alternatively or additionally be applied to the inner surface 42 of the first coverstock layer 22. For ease of illustration, the description and Figures refer to application to the second coverstock layer 26 only.

This pattern creates side anchor zones A, which substantially eliminate the delamination and creep associated with previously known laminates and which allows the laminate 20 to experience higher strains without creeping or delaminating. It has also been found that confining the first adhesive 70 to the edge areas 80 of the laminate structure 20 avoids impeding the extensibility of the laminate 20 and also avoids tears in the coverstock layers 22 and 26. Preferably, the first adhesive 70 is applied as a plurality of beads 68, as shown in the Figures. Preferably, the first adhesive 70 is a flexible adhesive with an amorphous and crystallizing component. Such a preferred adhesive is made by the Findley Adhesive Company under the designation H9224.

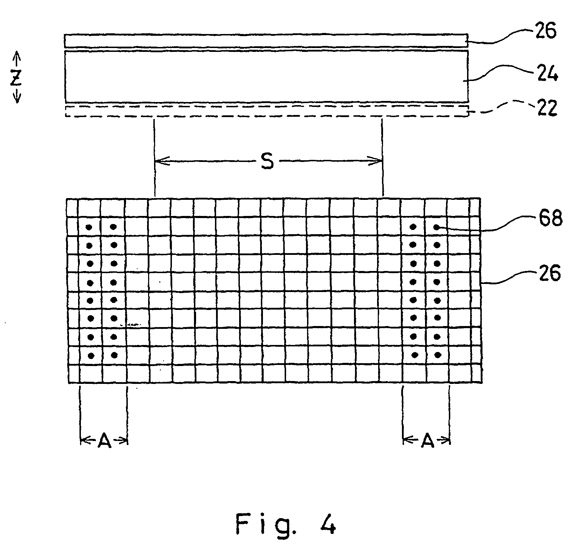

Figure 4 is a simplified view of the laminate 20 showing the locations of the side anchor zones A created by the beads 68, and the area of comparatively higher extensibility S that exists in the central area of the laminate 20. For ease of illustration, the second coverstock layer 26 is shown in phantom lines, and the elastomeric layer 24 is shown superposed on the second coverstock layer 26. The zones A depicted in the Figures are exemplary, and it will be understood by those of skill in the art that these side anchor zones A may accommodate varying design needs, for example with respect to exact location, width, and spacing, and are not limited to those shown in the Figures. Figure 4 also illustrates preferred positions of the beads 68 substantially within the apertures 32 of the elastomeric layer, which contributes to the integrity of the side anchor zones A for the reasons described more fully below.

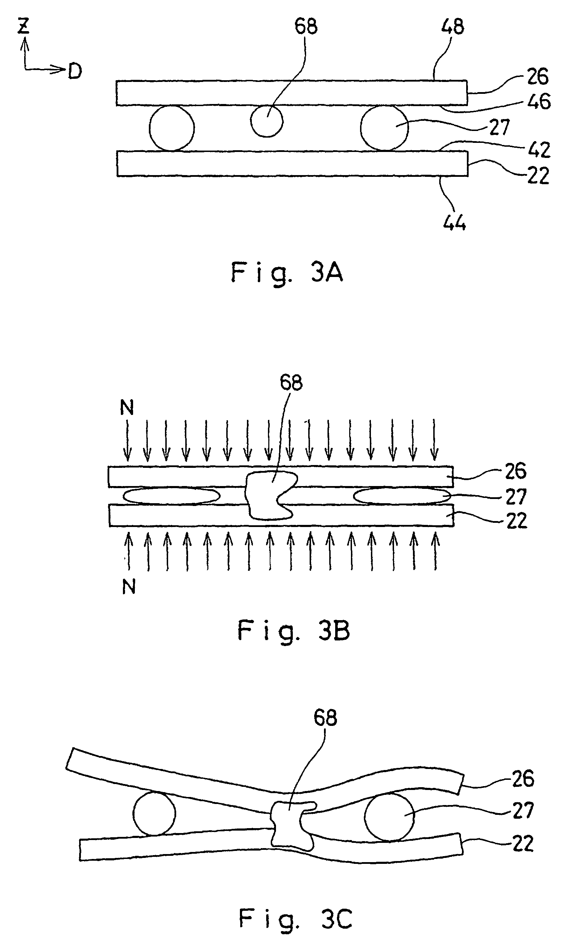

Upon application, each of the beads 68 is preferably absorbed in the z direction, i.e., into the thickness of, the second coverstock layer 26, where it encases the fibers of the coverstock layer in the area of application. Figure 3A is an expanded view of a portion of the laminate 20 showing the position of a glue bead 68 after initial application to the inner surface 46 of the second coverstock layer 26. Preferably, multiple beads 68 are applied so as to be continuous in the structural direction B. The beads 68 are preferably applied at a level of about 0.2 to about 1.4 mg per linear centimeter per bead (about 0.5 to about 3.5 milligrams per linear inch per bead), more preferably at about 0.6 mg per linear centimeter per bead (about 1.5 milligrams per linear inch per bead). Figure 3A also illustrates the preferred positions of the beads 68 substantially within the apertures 32 of the elastomeric layer. Another preferable application method is zone coating of beads 68.

Before the adhesive bead 68 has set, it is preferably nipped, whereby the portions of the beads 68 that show through the elastomeric apertures 32 come into contact with the inner surface 42 of the first coverstock layer 22. If the nip pressure N is sufficiently high, the adhesive of bead 68 will flow into the matrix of first coverstock layer 22. Figure 3B shows a typical position of a glue bead 68 during nipping. During nipping, the first and second strands 25 and 27 of the elastomeric layer 24 (only first strands 27 are shown in Figure 3B) also undergo compression. However, when the nipping pressure N is removed, strands 25 and 27 return to their original configurations.

After the beads 68 have set, the first and second coverstock layers 22 and 26 are locked due to the bonding formed by the adhesive encasement of the fibers in the first and second coverstock layers 22 and 26, and the continuous z-directional cohesive bond that forms through the scrim apertures 32, as shown in Figure 3C.

The laminate 20 includes a second adhesive 64 (not shown separately in Figures). The second adhesive 64 is preferably applied to the second surface 52 of the elastomeric layer 24, but may alternatively be applied to the first surface 50 of the elastomeric layer 24. The second adhesive 64 is applied in a spiral spray pattern 66, thereby forming bond points 67b that are more discrete than would be formed by a linear spray application. Without being bound by theory, it is believed that most of the second adhesive 64 so sprayed aligns in the structural direction D. Thus, it has been found that spiral spraying results in very good activation properties. As used herein, "activation" refers to the ability to stretch. The second spiral spray pattern 66 and the bond points 67b are shown in Figures 2A and 2B.

It has been found that spraying the layer of second adhesive 64 directly onto the second surface 52 of the elastomeric layer 24 is more preferable than applying the second adhesive 64 to the opposing (i.e., second) coverstock layer 26. This is because the second adhesive 64 tends to penetrate through any residual processing agents or oils that may remain on the surface of the elastomeric layer 24. Such residual materials, if left to remain on the elastomeric layer 24, may weaken the adhesive bonds and thus the laminate structure 20 over time. For example, if these residual materials are left intact, the bonds used to form the laminate 20 may weaken during the time interval prior to consumer purchase of the product.

Peel values for the laminate 20 in the spiral adhesive areas are typically higher when the spirals 66 are applied directly to the elastomeric layer 24 than to the opposing (i.e., second) coverstock layer 26. As used herein, the term "peel value" refers to the amount of force required to separate the two layers of coverstock material, 22 and 26, from each other. Higher peel values typically equate to less chance of delamination in use.

A third adhesive 60 (not shown separately in Figures) may also preferably be applied to the inner surface 42 of the first coverstock layer 22. Preferably, the third adhesive 60 is an elastomeric adhesive. In a manner similar to that described with reference to the second spiral adhesive application 66, the first adhesive 60 is preferably applied in a spiral spray pattern 62, thereby forming bond points 67a that are more discrete than would be formed by a linear spray application. Without being bound by theory, it is believed that most of the first adhesive 60 so sprayed aligns in the structural direction D. The third spiral spray pattern 62 is shown in Figures 2A and 2B.

Preferably, second and third adhesives 60 and 64 are the same elastomeric adhesive. A preferred adhesive for use in the second and third adhesive spiral sprays 62 and 66 is made by the Findley Adhesive Company under the designation H2120. Preferably, the add-on level for each of the second and third spiral sprays 62 and 66 is about 0.62 to about 1.86 milligrams per square centimeter (about 4 to about 12 milligrams per square inch), more preferably about 1.24 milligrams per square centimeter (about 8 milligrams per square inch).

For ease of manufacture, the laminate structure 20 as shown in Figure 2A may be formed from materials having a total width W, with a perforation line P provided at about the centerline. Thus, after bonding has been performed, the structure may be divided into two sections defined by widths of W1 and W2, shown after perforation in Figure 2B. The perforation line P provides easy separation where the laminate structure 20 will be incorporated into a garment having two side panels, for example a pull-on diaper. It has been found that W equal to about 126 mm and resulting W1 and W2 equal to about 63 mm are suitable for a diaper converting line.

The laminate structures 20 of the present invention are highly resistant to both shear and peel forces, and experience substantially no delamination and creep during use. The crystallizing adhesive (e.g., beads 68) at the edges 80 of the laminate structure 20 creates a zone of mechanical lockdown (i.e., side anchor zones A) in the linear region of the edges 80 of the laminate 20. The preferred adhesive pattern described herein permits the construction of a laminate 20 that can stretch up to 200% with no risk of creep of the elastomeric layer 24 substrate. Creep is measured using a test protocol of pre-stretching the laminate 20 to a 200% extension and returning it to 100% extension and holding for 24 hours in a 38° C test chamber. Success criteria is considered to be no movement at edges of the laminate 20 for all samples tested. This creep test simulates in-use fatigue by the shearing action of the elastomeric layer 24 during wear. The shearing action is induced by the movement of the wearer, which causes continuous elongation and relaxation cycles from approximately 24% to 150% strain over the wear period. Conventional bonds, such as those with elastic adhesives only, typically fail this creep test.

Raw materials and composites using previously known lamination technologies (for example elastic films, Lycra strands, conventional nonwoven coverstock materials, single adhesive systems) have all required trade offs in performance criteria, which has led to sacrifices in at least some of these performance criteria. The preferred laminate structures 20 are substantially free from delamination and creep while also possessing many desirable performance characteristics, without tradeoffs in any performance characteristic category.

For example, stress/strain characteristics of the laminate 20 preferred when incorporating the laminate into a typical disposable pull-on garment include a low application force (i.e. less than about 1.57 N per centimeter (about 400 grams per inch) at 200% extension, more preferably less than about 0.98 N per centimeter (about 250 grams per inch) at 200% extension), a high initial contractive force (i.e., greater than about 0.29 N per centimeter (about 75 grams per inch) at 50% extension), and a high sustained contractive force (i.e., greater than about 0.24 N per centimeter (about 60 grams per inch) at 50% extension, measurable for 12 hours at 38°C). The preferred laminate 20 further has high-speed activation properties (i.e., about 228.6 meter per minute (about 750 feet per minute)), and very good breathability characteristics (i.e., greater than about 4000 MVTR uniformly over entire surface, where MVTR (mass vapor transmission rate is calculated and expressed in $g/m^2/24$ hr. using the following formula:

$$MVTR = \frac{(\text{Final weight - initial weight}) \times 24.0}{\text{Area of sample in meters} \times 5.0 \text{ (time in chamber)}}.$$

Alternative embodiments for the laminate structure 20 can be formed by locating beads 68 uniformly across the structural direction D of the laminate. This structure is especially suited for uses in which activation is less demanding and thus the beads 68 need not be confined to the edge regions 30.

Another alternate embodiment provides that the beads 68 are applied so that they are discontinuous in the structural direction B. The beads may be located as a series of discrete dots. Such dots may be applied in any number of patterns with varying shape frequency of repetition and spacing both in the structural direction B and in the structural direction D.

In other alternative embodiments, the side anchoring may be achieved by means other than adhesive bead application. Side anchoring may be achieved by mechanical interlocking means such as selectively needle punching, sewing, or hydroentangling the edge regions of the coverstock layers to achieve interlocking of the edge regions 80 through the elastomeric layer apertures 32. Alternatively, selectively applying heat so that the edge regions 80 of the coverstock layers 22, 26 are fused together to form the side anchor zones A may be performed. Ultrasonic or thermal nipping may alternatively be used to selectively melt and lock the outer edges 80 to form the side anchor zones A. Such are given as examples of alternative preferred edge bonding means and it is to be understood that any suitable means known to one skilled in the art may by used in connection with the side anchoring of the laminate.

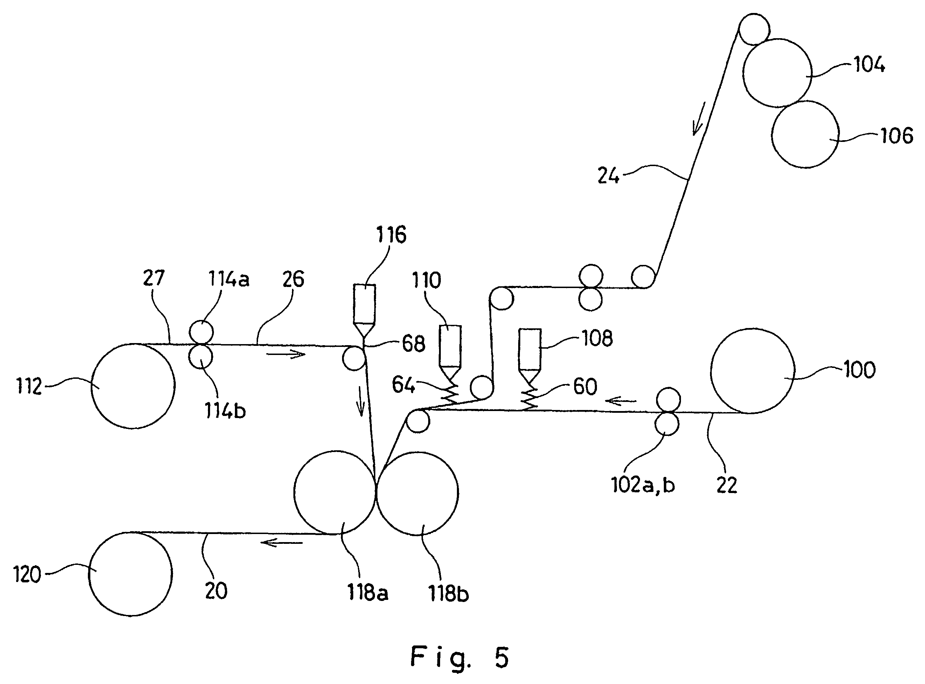

Figure 5 shows a preferred layout for a process for continuous manufacture of an edge bonded laminate structure 20 as described herein. The first coverstock layer 22 is unwound from a first unwind roll 100, preferably at a maximum of about 1 % strain and at a web speed of about 91.44 meter per minute (about 300 feet per minute). The first coverstock layer 22 may additionally be passed through first pair of tension rollers 102a, 102b. (It will be understood by those of skill in the art that various other, additional or alternative tension rolls or other processing equipment may be used in connection with the present invention without departing from its scope.) At approximately the same time, the elastomeric layer 24 is unwound from an elastomeric unwind roll 104, preferably at a maximum strain of about 1% and at a web speed of about 91.44 meter per minute (about 300 feet per minute). A release liner roll 106 that cooperates with the elastomeric unwind roll 104 may serve to take up a release liner (not shown) that may be juxtaposed with the elastomeric layer 24 on the elastomeric unwind roll 104 to facilitate elastomeric unwind, i.e., to prevent the elastomeric layer 24 from sticking or otherwise adhering to itself. Such a release liner will typically be discarded.

At the same time that the first coverstock layer 22 and the elastomeric layer 24 are being unwound, the second coverstock layer 26 is being unwound from second coverstock unwind roll 112, preferably at a maximum strain of about 1% and at a web speed of about 81.44 meter per minute (about 300 feet per minute.). The second coverstock layer 26 may additionally be passed through a second pair of tension rollers 114a, 114b. (It will be understood by those of skill in the art that various other, additional or alternative tension rolls or other processing equipment may be used in connection with the present invention without departing from its scope.)

At-least the first adhesive 70 is applied to the inner surface 46 of the second coverstock layer 26, preferably in the form of beads 68 (not shown in Figure 5) positioned at each of the outer edges 80 (not shown in Figure 5) of the second coverstock layer 26 and continuous in the structural direction B. The first adhesive 70 is supplied to a first adhesive applicator 116, which is generally located close enough to the second coverstock layer 26 to facilitate the proper adhesive application pattern. A bead 68 pattern as described above is preferred. The first adhesive 70 may alternatively or additionally be applied to the inner surface 42 of the first coverstock layer 22. For ease of illustration, the description and Figures refer to application to the second coverstock layer 26 only.

The elastomeric layer 24, the first coverstock layer 22 with the first adhesive 70 applied to the edges regions 80 thereof, and the second coverstock layer 26, are brought into contact with each other as they are passed between cooperating nipping rolls 118a and 118b. As described with reference to Figures 3A-3B, the nipping rolls 118 cause the contact between the coverstock layers 22 and 26 to join the elastomeric layer 24 therebetween, creating the side anchor zones A. Preferably the adhesive beads 68 show through the apertures 32 of the elastomeric layer 24, contacting the first coverstock layer 22 and also preferably causing the beads 68 to flow into the fiber matrix of the first coverstock layer 22 to create the cohesive z-directional bond. After nipping, the finished laminate material 20 may be rolled onto the rewind stand 120, where the adhesive is permitted to cure.

In another preferred embodiment, a second adhesive 64 is additionally provided to join at least the elastomeric layer 24 to one of the coverstock layers 22, 26. The second adhesive 64 may also join the first and second coverstock layers 22, 26 to each other. The second adhesive 64 is preferably applied directly to the second surface 52 (not shown in Figure 5) of the elastomeric layer 24 from the second adhesive applicator 110. The second adhesive 64 is supplied to the second adhesive applicator 110, which is generally located close enough to the elastomeric layer 24 to facilitate the proper adhesive application pattern. The second adhesive 64 is preferably applied in a spiral spray configuration at an add-on level of about 0.62 to about 1.86 milligrams per square centimeter (about 4 to about 12 milligrams per square inch), more preferably at about 1.24 milligrams per square centimeter (about 8 milligrams per square inch).

In still another preferred embodiment, a third adhesive 60 is additionally provided to join at least the elastomeric layer 24 to one of the coverstock layers 22, 26. The third adhesive 60 may also join the first and second coverstock layers 22, 26 to each other. The third adhesive 60 is preferably applied to the inner surface 42 of the first coverstock layer 22 (not shown in Figure 5). The third adhesive 60 is supplied to the third adhesive applicator 108, which is generally located close enough to the first coverstock layer 22 to facilitate the proper adhesive application pattern. The third adhesive 60 is preferably applied in a spiral spray configuration at an add-on level of about 0.62 to about 1.86 milligrams per square centimeter (about 4 to about 12 milligrams per square inch), more preferably about 1.24 milligrams per square centimeter (about 8 milligrams per square inch).

As will be understood by those of skill in the art, process variables such as web speed and adhesive flow rates may be altered. The adhesive application processes may be altered; also, any combination of the adhesives 60, 64 and 70 is possible. For example, gravure or screen printing can be used as the first adhesive applicator 116 to apply an adhesive bead 68 pattern. Additionally, meltblown adhesive, Nordson control coat, or any comparable fiberization process may be used instead of the spiral spray systems to apply the second and third adhesives 60 and 64. Numerous other medications are possible without departing from the scope of the present invention.

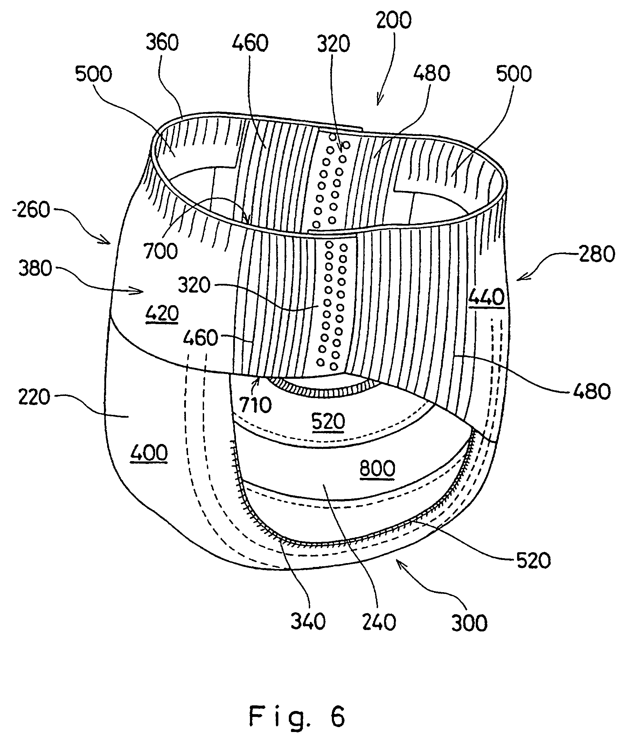

The laminate structure 20 can be incorporated into a variety of products wherein it is desired to provide at least one structural direction that is partially or entirely elastic along its length. Examples of such products include elastic diapers, tape diapers, incontinence products, bandages, body wraps and the like. One particularly preferred use of laminate structure 20 is as a side panel of a pull-on garment, for example the unitary pull-on diaper shown in Figure 6.

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). A "unitary" pull-on garment refers to pull-on garments which are formed of separate parts united together to form a coordinated entity, but the side panels are not separate elements joined to a separate chassis in that the side panels are formed by at least one layer which also forms the central panel or chassis of the garment (i.e., the garment does not require separately manipulative panels such as a separate chassis and separate side panels). The pull-on garment is also preferably "absorbent" to absorb and contain the various exudates discharged from the body. Exemplary is the unitary disposable absorbent pull-on garment, pull-on diaper 200, shown in Figure 6. As used herein, the term "pull-on diaper" refers to pull-on garments generally worn by infants and other incontinent individuals to absorb and contain urine and feces. It should be understood, however, that other pull-on garments such as training pants, incontinent briefs, feminine briefs, feminine hygiene garments or panties, and the like, are included herein.

Figure 6 is a perspective view of an exemplary pull-on diaper. The pull-on diaper 200 has an outer surface 220, an inner surface 240 opposed to the outer surface 220, a front region 260, a back region 280, a crotch region 300, and seams 320 which join together the front region 260 and the back region 280 to form leg openings 340 and a waist opening 360. A continuous belt 380 is formed about the waist opening 360. The continuous belt 380 acts to dynamically create fitment forces and to distribute the forces dynamically generated during wear. The pull-on diaper 200 thus preferably includes a chassis layer 400; a first belt layer 420; a second belt layer 440; an elastically extensible stretch panel positioned in each side panel of the front region 260 (front stretch panels 460), an elastically extensible stretch panel positioned in each side panel of the back region 280 (back stretch panels 480), and an elastic waist feature 500 positioned in both the front region 260 and the back region 280. The pull-on diaper 200 additionally comprises elastic leg features 520. Because the first belt layer 420 and the second belt layer 440 (the belt layers) are preferably nonwoven webs having the appearance of cloth and the chassis layer 400 is preferably a plastic film, the pull-on diaper 200 has a unique aesthetic feature in that it is perceived by caregivers and wearers to have a garment-like comfort and feel in the waist regions while having a perceived containment benefit in the crotch region. Such a pull-on diaper is disclosed in U.S. Patent No. 5,569,234 to Buell et al., which is incorporated by reference herein in its entirety.

The front stretch panels 460 and the back stretch panels 480 are unitary elements of the pull-on diaper 200 (i.e., they are not separately manipulative elements secured to the pull-on diaper, but rather are formed from and are extensions of one or more of the various layers (at least the belt layers 420, 440 and preferably also the chassis layer 400) of the pull-on diaper 200.) In a preferred embodiment, each stretch panel is formed by a portion of the chassis layer, a portion of the respective belt layer, an elastic panel member positioned between the chassis layer and the belt layer, and a portion of the barrier layer.

The belt 380 is elastically extensible in the side panels 460 and 480 to provide a more comfortable and contouring fit by initially conformably fitting the pull-on diaper 200 to the wearer and sustaining this fit throughout the time of wear well past when it has been loaded with exudates by distributing forces along both the waist and legs since the sides of the pull-on diaper 200 can expand and contract. The side panels 460 and 480 are extensible in at least one direction, preferably in a direction having a vector component in the lateral direction, more preferably in the lateral direction, to provide better fit. It should be noted, however, that the side panels 460 and 480 may be extensible in any other direction or in more than one direction. In addition, the side panels may have one or more discrete zones of extensibility.

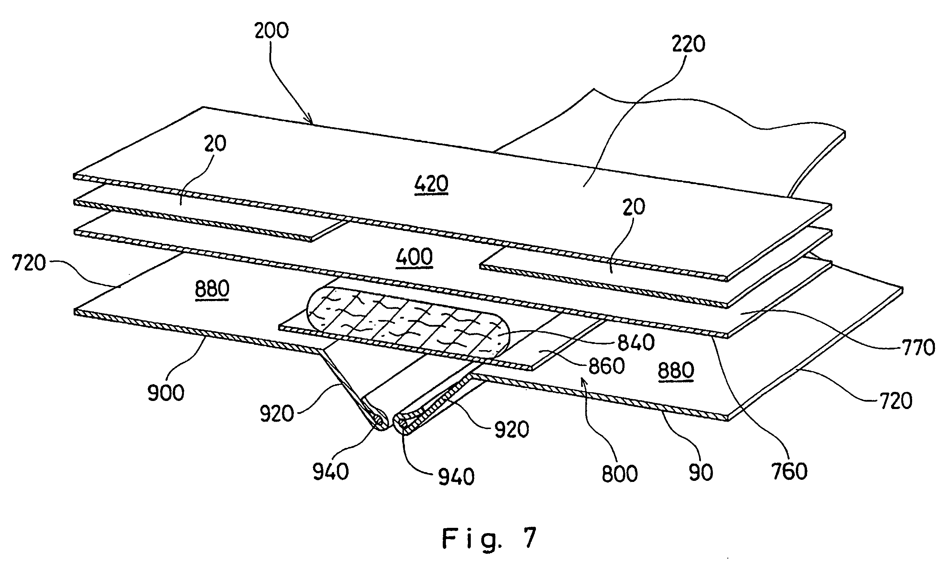

Elastically extensible stretch panels (front stretch panels 460 and back stretch panels 480) are formed in each side panel of both the front region 260 and the back region 280. As used herein, the term "panel" denotes an area of element of the pull-on diaper or belt. Figure 7 illustrated a sectional view of the front region 260 of the diaper 20. Each front stretch panel 460 at least comprises the portion of the first belt layer 420 in the side panel and a laminate member 20 according to the present invention joined thereto, and, in this particular illustration, the portion of the chassis layer 400 forming the side panel. Preferably, the laminate member 20 is positioned between the chassis layer 400 and the first belt layer 420, and more preferably extends longitudinally from the end edge 700, most preferably to the leg edge 710 (shown in Figure 6). Use of a laminate structure 20 according to the present invention is highly preferred.

Although Figure 7 shows only a sectional view of the front region 260 of the pull-on garment 200, the back region 280 is similarly constructed. Each rear stretch panel 480 at least comprises the portion of the second belt layer 440 in each side panel and a (rear) laminate member 20' (not shown) joined thereto, and, in this particular embodiment, the portion of the chassis layer 400 forming the side panel. Preferably, the (rear) laminate member 20' is positioned between the chassis layer 400 and the second belt layer 440, and more preferably extends longitudinally from the end edge 700' (not shown), most preferably to the leg edge 710' (not shown).

As shown in Figure 6, the chassis layer 400 is shown to form the primary strata or layer of the pull-on diaper 200 and has an inner surface 760 and an outer surface 770. The first belt layer 420 is positioned on the outer surface 770 of the chassis layer 400 to form the outer surface 220 of the pull-on diaper 200 in the front region 260. The laminate members 20 are preferably positioned between the first belt layer 420 and the chassis layer 400. The topsheet 800 preferably comprises a liquid pervious primary layer 860 and two barrier layers 880. The barrier layers 880 extend laterally outwardly from the primary layer 860 to the side edges 720. Each barrier layer 880 comprises a flap portion 900 and a stand-up portion 920 to stand up away from the surface of the primary layer 860 to form a barrier or wall in use. The flap portion 900 extends laterally outwardly from the stand-up portion 920 to the side edge 720. The absorbent core 840 is preferably positioned between the primary layer 860 and the chassis layer 400. The construction of the back region is preferably identical to the construction of the front region 260.

The laminate member 20 can be operatively joined in the stretch panels 460 to the chassis layer 400, the belt layers 380, or both, using either an intermittent bonding configuration or a substantially continuous bonding configuration. As used herein, an "intermittently" bonded laminate web means a laminate web wherein the plies are initially bonded to one another at discrete spaced apart points or a laminate web wherein the plies are substantially unbounded to one another at discrete spaced apart areas. Conversely, a "substantially continuously" bonded laminate web means a laminate web wherein the plies are initially bonded substantially continuously to one another throughout the areas of interface. Such methods are described, e.g., in U.S. Patent 3,860,003 entitled "Contractible Side Portions For A Disposable Diaper" issued to Buell on January 14, 1975, WO 95/03765, "Web Materials Exhibiting Elastic-like Behavior", The Procter & Gamble Company, published February 9, 1995, each of which is incorporated herein by reference. Alternatively, the laminate member and any other components of the stretch panel may be intermittently or continuously bonded to one another using heat bonding, pressure bonding, ultrasonic bonding, dynamic mechanical bonding, or any other method as is known in the art.

It has been found that the extension characteristics including the extension forces, extension modulus, and available stretch (extension); and the contractive forces and rate of contraction of the stretch panels are important considerations in the performance of both the stretch panels and the pull-on diaper. The extension properties and fitment forces give the applicator and the wearer the overall perceived "stretchiness" during use. They also effect the ability of the applicator to achieve a suitable degree of application stretch (i.e., for a "normally" perceived tensioning of the diaper during application, the total amount of resultant stretch is that desired to achieve/maintain good conformity of fit). A stretch panel with a relatively high extension modulus/force can cause red marking on the wearer's skin while a relatively low extension modulus/force can cause sagging/slipping on the wearer. Stretch panels having too little available stretch may not achieve a suitable level of body conformity and may contribute in making the diaper uncomfortable to wear and hard to put on. Stretch panels with very low contractive forces (or excessive elastic creep, excessive elastic force relaxation, or excessive inelastic "set") may not stay in place on the wearer and may tend to sag/slip on the wearer resulting in poor fit and containment. Incorporation of laminate structures 20 of the present invention into the side panels 460 and 480 of the pull-on diaper 20 achieve a high level of body conformity while substantially eliminating sag and slip.

The pull-on diaper 200 can be applied by a caregiver or be self-applied by the wearer. Typically, the waist opening 360 will be expanded to allow the wearer to insert one foot into one of the leg openings 340. The other foot is then inserted into the other leg opening 340. The pull-on diaper 200 is then pulled up over the torso of the wearer into its wearing position. The force wall created by side panels 460, 480 that incorporate the laminate structures 20 of the present invention especially assists in self application of the pull-on diaper by forcing the product to be pulled up over the buttocks rather than further expanding. The pull-on diaper is then worn and can contain and hold discharged body exudates. The pull-on diaper is removed from the wearer by either pulling it back down over the legs or tearing the portions of the pull-on diaper adjacent the seams.

For form-fitting garments such as the pull on diaper 200, the breathability and the circumferential forces of the garment are important to performance, especially in hot and humid conditions. When an absorbent article is placed on a wearer, the skin is occluded by the materials making up the garment, which prevents evaporation and resultant cooling of the occluded area. This in turn results in less comfort for the wearer. Thus, sufficient moisture vapor transmission in the side panels is a desirable performance characteristic. Exemplary side panels 460 and 480 having a desirable weighted average mass vapor transmission rate include a laminate structure 20 according to the present invention.

**Claims**

1. A method for forming a laminate structure (20) elastically extensible in at least one direction, comprising the steps of:

    providing a first coverstock layer (22) having an inner (42) and an outer (44) surface;

    providing a second coverstock layer (26) having an inner (46) and an outer (48) surface;

    providing an elastomeric layer (24) between the first and the second coverstock layers; and

    either applying at least a first adhesive (70) to edge regions (80) of the inner surface of at least one of the coverstock layers (22, 26) to provide side anchor zones (A) along edge regions (80) of the laminate structure (20); or performing side anchoring by sewing, heat sealing, ultrasound bonding, needle punching, **characterized in that** said laminate (20) further comprises a second adhesive (60), said second adhesive (60) being applied in a spiral spray pattern (66).

2. The method of claim 1 further comprising the step of applying a pressure to the combination of the first coverstock layer (22), the elastomeric layer (24), and the second coverstock layer (26) sufficient to cause the first adhesive (70) to flow into the other of the coverstock layers (22, 26) to create a cohesive bond between the first and the second coverstock layers (22, 26).

3. The method of claim 1 further comprising the step of applying a third adhesive (60) to join at least one of the coverstock layers (22, 26) to at least the elastomeric layer (24).

4. The method of claim 3 wherein the first adhesive (70) is applied as a plurality of discrete beads (68).

5. The method of claim 4 wherein each of the second adhesive (64) and the third adhesive (60) is an elastomeric adhesive applied in a spiral spray pattern (62).

6. The method of claim 1
    wherein said elastomeric layer (24) is an apertured elastomeric having a first surface (50) that faces the inner surface (42) of the first coverstock layer (22) and a second surface (52) that faces the inner surface of the second coverstock layer; and
    wherein the first and second coverstock layers (22,26) are joined by cohesive bonds that form through the apertures (32) of the elastomeric layer (24).

**Patentansprüche**

1. Verfahren zum Bilden einer Laminatstruktur (20), die in zumindest einer Richtung elastisch dehnbar ist, umfassend die Schritte:

    Bereitstellen einer ersten Deckmaterial-Lage (22) mit einer inneren (42) und einer äußeren (44) Oberfläche;
    Bereitstellen einer zweiten Deckmaterial-Lage (26) mit einer inneren (46) und einer äußeren (48) Oberfläche;
    Bereitstellen einer elastischen Lage (24) zwischen den ersten und zweiten Deckmaterial-Lagen; und
    entweder Aufbringen zumindest eines ersten Klebstoffes (70) auf die Kantenbereiche (80) der inneren Oberfläche von zumindest einer der Deckmaterial-Lagen (22, 26), um eine Seitenverankerungszone (A) entlang der Kantenbereiche (80) der Laminatstruktur (20) zu bilden; oder
Durchführen der Seitenverankerung durch Nähen, Heißsiegeln, Ultrasthallbinden, Nadelstanzen,

**dadurch gekennzeichnet, dass** das Laminat (20) weiterhin einen zweiten Klebstoff (60) umfasst, wobei der zweite Klebstoff (60) in einem spiralförmigen Sprühmuster (66) aufgetragen ist.

2. Verfahren nach Anspruch 1, ferner umfassend den Schritt, einen Druck auf die Kombination der ersten Deckmaterial-Lage (22), der elastischen Lage (24) und der zweiten Deckmaterial-Lage (26) aufzubringen, der ausreicht zu veranlassen, dass der erste Klebstoff (70) in die andere der Deckmaterial-Lagen (22, 26) fließt, um eine kohäsive Bindung zwischen den ersten und zweiten Deckmaterial-Lagen (22, 26) zu bilden.

3. Verfahren nach Anspruch 1, ferner umfassend den Schritt, einen dritten Klebstoff (60) aufzubringen, um zumindest eine der Deckmaterial-Lagen (22, 26) mit zumindest der elastischen Lage (24) zu verbinden.

4. Verfahren nach Anspruch 3, wobei der erste Klebstoff als Vielzahl von einzelnen Tropfen (68) aufgebracht wird.

5. Verfahren nach Anspruch 4, wobei sowohl der zweite Klebstoff (64) als auch der dritte Klebstoff (60) ein elastischer Klebstoff ist, der in einem spiralförmigen Sprühmuster (62) aufgebracht ist.

6. Verfahren nach Anspruch 1, wobei die elastische Lage (24) ein mit Öffnungen versehenes Elastomer ist, das eine erste Oberfläche (50), die der inneren Oberfläche (42) der ersten Deckmaterial-Lage (22) zugewandt ist, und eine zweite Oberfläche (52) aufweist, die der inneren Oberfläche der zweiten Deckmaterial-Lage zugewandt ist, und wobei die erste und zweite Deckmaterial-Lage (22, 26) durch kohäsive Eindungen verbunden sind, die sich durch die Öffnungen (32) der elastischen Lage (24) bilden.

## Revendications

1. Procédé pour produire une structure de stratifié (20) extensible élastiquement dans au moins une direction, comprenant les étapes consistant à :

   fournir une première couche de matériau de recouvrement (22) comportant une surface intérieure (42) et une surface extérieure (44) ;
   fournir une deuxième couche de matériau de recouvrement (26) comportant une surface intérieure (46) et une surface extérieure (48) ;
   fournir une couche élastomère (24) entre les première et deuxième couches de matériau de recouvrement ; et soit appliquer au moins un premier adhésif (70) sur des régions de bord (80) de la surface intérieure d'au moins une des couches de matériau de recouvrement (22, 26) afin de fournir des zones de fixation (A) latérales le long des régions de bord (80) de la structure de stratifié (20),
   soit réaliser une fixation latérale par couture, thermoscellage, liaison par ultrasons, aiguilletage,

   **caractérisé en ce que** ledit stratifié (20) comprend, en outre, un deuxième adhésif (60), ledit deuxième adhésif (60) étant appliqué selon un motif de pulvérisation en spirale (66).

2. Procédé selon la revendication 1, comprenant, en outre, l'étape consistant à appliquer une pression sur la combinaison d'éléments formée par la première couche de matériau de recouvrement (22), la couche élastomère (24), et la deuxième couche de matériau de recouvrement (26), suffisante pour amener le premier adhésif (70) à s'écouler d'une des deux couches de matériau de recouvrement (22, 26) dans l'autre, afin d'engendrer une liaison cohésive entre les première et deuxième couches de matériau de recouvrement (22, 26).

3. Procédé selon la revendication 1, comprenant, en outre, l'étape consistant à appliquer un troisième adhésif (60) afin de réunir au moins une des couches de matériau de recouvrement (22, 26) au moins à la couche élastomère (24).

4. Procédé selon la revendication 3, dans lequel le premier adhésif (70) est appliqué à la manière d'une pluralité de dépôts (68) distincts.

5. Procédé selon la revendication 4, dans lequel chacun des deuxième (64) et troisième (60) adhésifs est un adhésif élastomère appliqué selon un motif de pulvérisation en spirale (62).

6. Procédé selon la revendication 1, dans lequel ladite couche élastomère (24) est un matériau élastomère perforé comportant une première surface (50) qui fait face à la surface intérieure (42) de la couche de matériau de recouvrement (22), et une deuxième surface (52) qui fait face à la surface intérieure de la deuxième couche de matériau de recouvrement; et dans lequel les première et deuxième couches de matériau de recouvrement (22, 26) sont réunies par des liaisons cohésives qui se forment à travers les orifices (32) de la couche élastomère (24).

Fig. 1

Fig. 2A

Fig. 2B

Fig. 3A

Fig. 3B

Fig. 3C

Fig. 4

Fig. 5

Fig. 6

Fig. 7